# EUROPÄISCHE PATENTSCHRIFT

(11) **EP 1 776 044 B1**
(45) Veröffentlichungstag und Bekanntmachung des Hinweises auf die Patenterteilung: **22.12.2010**
(21) Anmeldenummer: 05767981.3
(22) Anmeldetag: 11.07.2005
(51) Int. Cl.: A61B 6/00, H05G 1/02, G01T 1/16

(54) **GERÄT ZUR MEDIZINISCHEN BILDGEBUNG**
MEDICAL IMAGING EQUIPMENT
APPAREIL D'IMAGERIE MEDICALE

(30) Priorität: 15.07.2004 DE 102004034239
(43) Veröffentlichungstag der Anmeldung: 25.04.2007
(73) Patentinhaber: SIEMENS AKTIENGESELLSCHAFT, 80333 München (DE)
(72) Erfinder: HOLLER, Wolfgang, 91052 Erlangen (DE); RAMSAUER, Martin, 90602 Pyrbaum (DE); NIEWALDA, Gregor, 91054 Erlangen (DE)
(86) Internationale Anmeldenummer: PCT/EP2005/053312
(87) Internationale Veröffentlichungsnummer: WO 2006/005744

(56) Entgegenhaltungen:
- DE-A1- 3 319 622
- US-A- 4 104 529
- US-A- 5 170 419
- US-B1- 6 304 627

## Beschreibung

Die vorliegende Erfindung betrifft ein Gerät zur medizinischen Bildgebung mit den Merkmalen des Oberbegriffes des unabhängigen Anspruches 1 und insbesondere ein Gerät zur Gewinnung von Mammografiebildern.

Bei der Mammografie handelt es sich um eine Röntgenuntersuchung der weiblichen Brust, die mittels Geräten zur medizinischen Bildgebung zur Gewinnung von Mammografiebildern ausgeführt wird. Derartige Geräte weisen üblicherweise eine Strahlungsquelle für Röntgenstrahlung auf. Mittels der Röntgenstrahlung wird die zu untersuchende weibliche Brust durchleuchtet und auf einem im Strahlengang unterhalb der weiblichen Brust angeordneten Röntgenfilm ein Durchleuchtungsbild gewonnen. Während der Untersuchung wird die weibliche Brust üblicherweise zwischen einer Kompressionsplatte und einem Objekttisch gehalten.

Die Verwendung von Röntgenfilmen hat den Vorteil, dass sie eine technisch relativ ausgereifte und zumindest in der Anschaffung relativ kostengünstige Lösung darstellt und gleichzeitig eine dauerhafte Archivierung eines gewonnenen Durchleuchtungsbildes mittels des Röntgenfilmes möglich ist.

Ein weiterer Vorteil der Verwendung von Röntgenfilmen ist, dass Röntgenfilme eine sehr große Empfangsfläche von typischerweise 18x24 cm oder 24x30 cm und eine relativ hohe Ortsauflösung von etwa 14 Lp/mm (Lp = Linienpaare) aufweisen, wodurch eine hochauflösende Gesamtaufnahme der weiblichen Brust in einer einzigen Messung möglich ist.

An Stelle des nur einmal verwendbaren Röntgenfilmes ist die Verwendung von CCD-Sensoren (Charge-Coupled-Device) bekannt, welche an Stelle des Röntgenfilmes in ein Mammografiegerät eingesetzt werden können.

Um eine einfache Adaption bei vorhandenen Geräten zu gewährleisten, ist der CCD-Sensor häufig in eine Halterung mit der Form einer gewöhnlichen Röntgenfilmkassette integriert.

Bei CCD-Sensoren handelt es sich um elektronische Bauteile, die zur ortsauflösenden Messung von Strahlung, insbesondere Röntgenstrahlung geeignet sind und in der Regel aus einer Matrix von strahlungsempfindlichen Zellen, die auch Pixel genannt werden, bestehen.

Vorteilhaft an der Verwendung von CCD-Sensoren ist, dass moderne CCD-Sensoren einerseits eine Auflösung von zwischen 10 und 20 Lp pro Millimeter aufweisen, welche die Auflösung von Röntgenfilmen zum Teil übertrifft und die Bilder andererseits sofort bereitgestellt und digital bearbeitet werden können. Somit sind CCD-Sensoren (anders als Röntgenfilme) zur Gewinnung von Echtzeitbildern (beispielsweise während einer Biopsie) geeignet.

Nachteilig an der Verwendung von CCD-Sensoren ist, dass bekannte CCD-Sensoren mit der erforderlichen hohen Auflösung eine Empfangsfläche aufweisen, die wesentlich kleiner als die Empfangsfläche von Röntgenfilmen ist. Daher eignen sich hochauflösende CCD-Sensoren derzeit lediglich für Detailaufnahmen der weiblichen Brust.

Weiter ist im Rahmen der FFDM (Full-Field-Digital-Mammographie) die Verwendung von niedrigauflösenden Digitaldetektoren bekannt.

Die für die FFDM verwendeten Digitaldetektoren weisen eine Auflösung von derzeit typischerweise 5 bis 10 Lp pro Millimeter und damit eine geringere Auflösung als Röntgenfilme auf. Dafür sind Empfangsflächen realisierbar deren Größe ähnlich der Größe der Empfangsflächen konventioneller Röntgenfilme ist. Mittels der FFDM Detektoren ist es daher möglich, mit einer Aufnahme ein Gesamtbild der weiblichen Brust zu erstellen.

Der entscheidende Vorteil der FFDM Detektoren liegt somit darin, dass die Bilder in Echtzeit zur Verfügung stehen, digital bearbeitet werden können und die Empfangsflächen relativ groß sind. Nachteilig ist die derzeit relativ geringe Auflösung.

Alternativ zu FFDM Detektoren ist zur Gewinnung von Mammografiebildern auch die Verwendung von digitaler Lumineszenzradiografie mit Speicherfolientechnik bekannt. Die mit dieser Technik derzeit erzielbare Auflösung beträgt etwa 8 Lp/mm.

Um die Vorteile beispielsweise eines FFDM Detektors mit dem Vorteil eines Röntgenfilms kombinieren zu können, sind Geräte zur medizinischen Bildgebung zur Gewinnung von Mammografiebildern bekannt, die zwei Empfangsflächen für Röntgenstrahlung aufweisen.

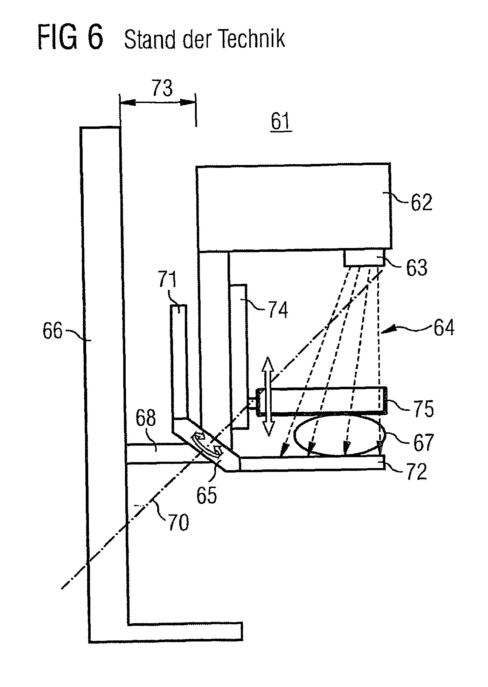

Ein entsprechendes Gerät mit zwei Empfangsflächen wird im Folgenden unter Bezugnahme auf Fig. 6 näher beschrieben.

Das vorbekannte Gerät zur medizinischen Bildgebung 61 zur Gewinnung von Mammografiebildern weist einen Kopf 62 mit einer Strahlungsquelle 63 zur Emission von Röntgenstrahlung 64 sowie eine Empfangseinrichtung 65 auf.

Sowohl der Kopf 62 als auch die Empfangseinrichtung 65 werden von einer Tragsäule 66 getragen, die in der Regel an einem Bodenstativ befestigt ist. Die Tragsäule 66 kann zusätzlich an der Decke eines Raumes befestigt sein.

In dem in Fig. 6 gezeigten Beispiel weist die Empfangseinrichtung 65 eine erste Empfangsfläche 71 in Form eines Halters für Röntgenfilme sowie eine zweite Empfangsfläche 72 in Form eines großflächigen aber niedrigauflösenden Detektors für FFDM Aufnahmen auf.

Die beiden Empfangsflächen 71 und 72 sind zueinander im rechten Winkel angeordnet und werden über eine an der Tragsäule 66 befestigte Halterung 68 von einem Träger 65 der Empfangseinrichtung getragen. Die beiden Empfangsflächen 71 und 72 können durch eine manuelle Drehung des Trägers um eine Drehachse 70 abwechselnd in eine Messstellung geschwenkt werden. Dabei schließt die Drehachse zu dem Strahlengang der von der Röntgenquelle 63 emittierten Röntgenstrahlung 64 einen Winkel von im Wesentlichen 45° ein.

Der Winkel von 45° zwischen Drehachse 70 und Strahlengang der Röntgenstrahlung 64 stellt in Verbindung mit den zueinander im 90° Winkel angeordneten Empfangsflächen sicher, dass nach einer Verschwenkung des Trägers 65 um die Drehachse 70 eine der Empfangsflächen 71 außerhalb des Strahlenganges parallel zum Strahlengang und die andere Empfangsfläche 72 innerhalb des Strahlenganges senkrecht zum Strahlengang der Röntgenstrahlung 64 angeordnet ist.

Im Strahlengang zwischen einer jeweiligen in Messstellung befindlichen Empfangsfläche 71 bzw. 72 und der Strahlungsquelle 64 ist ein Messbereich zum Anordnen eines Messobjektes 67 vorgesehen.

Weiter ist im Strahlengang oberhalb des Messobjektes 67 eine für die jeweilige Messstrahlung durchlässige Kompressionsplatte 75 vorgesehen.

Die Kompressionsplatte 75 wird von einer Kompressionseinrichtung 74 des vorbekannten Gerätes getragen. Durch eine von der Kompressionseinrichtung 74 bewirkte Vertikalbewegung der Kompressionsplatte 75 ist es möglich, das Messobjekt 67 zwischen der Kompressionsplatte 75 und einer von der jeweiligen Empfangsfläche 71 bzw. 72 gebildeten Auflagefläche zu komprimieren.

Bei dem vorbekannten Gerät ist es nachteilig, dass es aufgrund der ausladenden Schwenkbewegung der von dem Träger 65 gehaltenen Empfangsflächen 71 und 72 einen hohen Platzbedarf 73 aufweist.

Weiter ist das Herstellen einer entsprechend drehbaren mechanischen Verbindung zu den jeweiligen Empfangsflächen 71, 72 mit der auf dem Medizinsektor erforderlichen Genauigkeit technisch sehr anspruchsvoll und damit teuer.

Die Druckschrift DE 3319622 offenbart ein Gerät zur medizinischen Bildgebung mit den Merkmalen des Oberbegriffes des unabhängigen Anspruches 1.

Ausgehend davon ist es Aufgabe der vorliegenden Erfindung, ein Gerät zur medizinischen Bildgebung mit den Merkmalen des Oberbegriffes des unabhängigen Anspruches 1 zur Verfügung zu stellen, welches einen platzsparenden und mechanisch einfachen Aufbau aufweist und besonders robust ist.

Die Aufgabe wird bei einem Gerät zur medizinischen Bildgebung mit den Merkmalen des Oberbegriffes des unabhängigen Anspruches 1 durch die Merkmale des kennzeichnenden Teils des unabhängigen Anspruches 1 gelöst.

Vorteilhafte Ausgestaltungen finden sich in den Unteransprüchen.

Erfindungsgemäß weist ein Gerät zur medizinischen Bildgebung eine Strahlungsquelle zur Emission einer Messstrahlung, eine Empfangseinrichtung zum Empfangen der von der Strahlungsquelle emittierten Messstrahlung, sowie einen Messbereich zum Anordnen eines Messobjektes, der in einem Strahlengang der Messstrahlung zwischen Strahlungsquelle und Empfangseinrichtung angeordnet ist, auf, wobei die Empfangseinrichtung einen um eine Drehachse schwenkbaren Träger aufweist, der wenigstens zwei Empfangsflächen für die Messstrahlung trägt und die Empfangsflächen abwechselnd in eine Messstellung bewegen kann, wobei die Empfangsflächen parallel zur Drehachse des Trägers angeordnet sind und die Drehachse des Trägers zu dem Strahlengang der Messstrahlung senkrecht steht. Das Gerät ist dadurch gekennzeichnet, dass die Drehachse bezüglich dem Messbereich und der sich geweils in der Messstellung befindlichen Empfangsfläche in die der Strahlungsquelle abgewandte Richtung versetzt ist und die Empfangsflächen der Drehachse abgewandt sind.

Da erfindungsgemäß die Empfangsflächen im Wesentlichen parallel zur Drehachse des Trägers angeordnet sind und die Drehachse des Trägers zu dem Strahlengang der Messstrahlung im Wesentlichen senkrecht steht, führt ein Verschwenken des Trägers zu einer Bewegung, die in horizontaler Richtung nicht mehr Raum einnimmt als der in Messstellung befindliche Träger mit den von dem Träger gehaltenen Empfangsflächen. Außerdem ist es so möglich, den Träger als gemeinsames Gehäuse für die Empfangsflächen auszugestalten. Somit weist das erfindungsgemäße Gerät einen platzsparenden Aufbau auf. Weiter ist die mechanische Anbindung des Trägers an das Gerät besonders einfach und robust bereitstellbar.

Vorzugsweise schließen die Drehachse mit dem Strahlengang der Messstrahlung einen Winkel von zwischen 80° und 100°, vorzugsweise zwischen 85° und 95°, bevorzugt zwischen 88° und 92° und besonders bevorzugt von 90° ein.

Gemäß einer bevorzugten Ausführungsform ist die Drehachse zwischen den Empfangsflächen angeordnet.

Es ist vorteilhaft, wenn die Empfangsflächen von einer senkrechten Projektion der Drehachse jeweils im Wesentlichen halbiert werden.

Bei einer derartigen Anordnung der Drehachse und der Empfangsflächen wird zum Einen sichergestellt, dass die jeweilige Empfangsfläche nach dem Drehen des Trägers immer mittig zur Drehachse in der jeweiligen Messstellung zum Liegen kommt. Dies erlaubt eine intuitive und damit einfache Bedienung des Gerätes. Weiter können so Fehler aufgrund einer falschen Einschätzung der räumlichen Lage der Empfangsfläche vermieden werden. Zudem wird so sichergestellt, dass die Drehbewegung des Trägers möglichst wenig ausladend ist.

Bevorzugt ist die Drehachse zentral im Strahlengang der Messstrahlung angeordnet.

Besonders vorteilhaft ist es, wenn die Drehachse im geometrischen Schwerpunkt eines vom Träger für die Empfangsflächen umschlossenen Volumens angeordnet ist.

Bei einer Anordnung der Drehachse im geometrischen Schwerpunkt eines von dem Träger für die Empfangsflächen umschlossenen Volumens wird sichergestellt, dass der Träger beim Schwenken eine möglichst kleine Umlaufbahn aufweist. Weiter ist der Verlauf einer Schwenkbewegung um eine derartige Drehachse für einen Benutzer besonders gut vorhersehbar.

Vorzugsweise weist der Träger gleichzeitig eine Ablagefläche für das Messobjekt auf.

Somit kann die Oberfläche des Trägers als untere Kompressionsfläche für die Kompression der weiblichen Brust während einer Mammografieaufnahme dienen. Dieses reduziert die Anzahl der erforderlichen Bauteile und damit die Kosten des erfindungsgemäßen Gerätes.

Vorzugsweise ist zwischen den Empfangsflächen eine vom Träger getragene Kontrolleinrichtung zur Kontrolle der empfangenen Messstrahlung vorgesehen.

Durch die Verwendung einer vom Träger getragenen Kontrolleinrichtung zur Kontrolle der empfangenen Messstrahlung ist es möglich, eine gemeinsame Kontrolleinrichtung für verschiedene Empfangsflächen zu verwenden, wodurch die Anzahl der erforderlichen Bauteile reduziert wird. Die Kontrolleinrichtung kann beispielsweise einer automatischen Belichtungszeitregelung dienen.

Bevorzugt trägt der Träger zusätzlich wenigstens eine Abschirmung, die für nicht in der Messstellung befindliche Empfangsflächen im Strahlengang der Messstrahlung angeordnet ist.

Durch die Abschirmung der nicht in der Messstellung befindlichen Empfangsflächen kann verhindert werden, dass Röntgenstrahlung an einer der Strahlungsquelle abgewandten Seite des Trägers heraustritt und Körperteile des Patienten durchstrahlt, die nicht Gegenstand der Untersuchung sind. Hierdurch kann die Strahlenbelastung des Patienten verringert werden. Außerdem wird eine unbeabsichtigte Belichtung der nicht in Messstellung befindlichen Empfangsfläche sowie eine Strahlenbelastung dieser Empfangsfläche unterbunden.

Gemäß einer bevorzugten Ausführungsform trägt der Träger zwei im Wesentlichen parallele Empfangsflächen für die Messstrahlung.

Ein derartiger Aufbau ist mit einer äußerst kompakten Bauform realisierbar.

Gemäß einer weiteren bevorzugten Ausführungsform trägt der Träger drei Empfangsflächen für die Messstrahlung, wobei benachbarte Empfangsflächen miteinander einen Winkel von im Wesentlichen 60° einschließen.

Hierdurch können drei Empfangsflächen in einer kompakten Bauform realisiert werden.

Gemäß einer alternativen Ausführungsform trägt der Träger vier Empfangsflächen für die Messstrahlung, wobei benachbarte Empfangsflächen miteinander einen Winkel von im Wesentlichen 90° einschließen.

Dieser Aufbau erlaubt die Verwendung von vier Empfangsflächen mit Beibehaltung einer kompakten Bauform.

Vorzugsweise ist die von der Strahlungsquelle emittierte Messstrahlung eine Röntgenstrahlung.

Bevorzugt ist wenigstens eine der Empfangsflächen ein Festkörperdetektor für Röntgenstrahlung und vorzugsweise ein CCD-Sensor.

Es ist vorteilhaft, wenn wenigstens eine der Empfangsflächen ein Röntgenfilm ist.

Vorzugsweise ist wenigstens eine der Empfangsflächen eine Lumineszenz-Radiographie-Folie. Derartige Folien werden auch als "Speicherfolien" bezeichnet.

Besonders vorteilhaft ist es, wenn das Gerät zur medizinischen Bildgebung zur Gewinnung von Mammografiebildern ausgebildet ist.

Im Folgenden wird die vorliegende Erfindung unter Bezugnahme auf die beigefügten Zeichnungen im Detail erläutert. In den Zeichnungen, in denen gleiche Bezugszeichen gleiche Elemente in den Ansichten bezeichnen, zeigt
Fig. 1A schematisch eine Seitenansicht eines Gerätes zur medizinischen Bildgebung zur Gewinnung von Mammografiebildern gemäß einer ersten besonders bevorzugten Ausführungsform der vorliegenden Erfindung;
Fig. 1B schematisch eine Ansicht von vorn auf das erfindungsgemäße Gerät zur medizinischen Bildgebung von Fig. 1A;
Figs. 2A, 2B, 2C schematisch eine Schnittansicht durch einen Träger einer erfindungsgemäßen Empfangseinrichtung, wobei die verschiedenen Figuren verschiedene Drehzustände zeigen;
Fig. 3 schematisch eine Schnittansicht durch eine alternative Ausführungsform einer Empfangseinrichtung des erfindungsgemäßen Gerätes zur medizinischen Bildgebung;
Fig. 4 schematisch eine Schnittansicht durch einen Träger einer erfindungsgemäßen Empfangseinrichtung gemäß einer zweiten bevorzugten Ausführungsform der vorliegenden Erfindung;
Fig. 5 schematisch eine Schnittansicht durch einen Träger einer erfindungsgemäßen Empfangseinrichtung gemäß einer dritten bevorzugten Ausführungsform der vorliegenden Erfindung; sowie
Fig. 6 schematisch ein Gerät zur medizinischen Bildgebung zur Gewinnung von Mammografiebildern nach dem Stand der Technik.

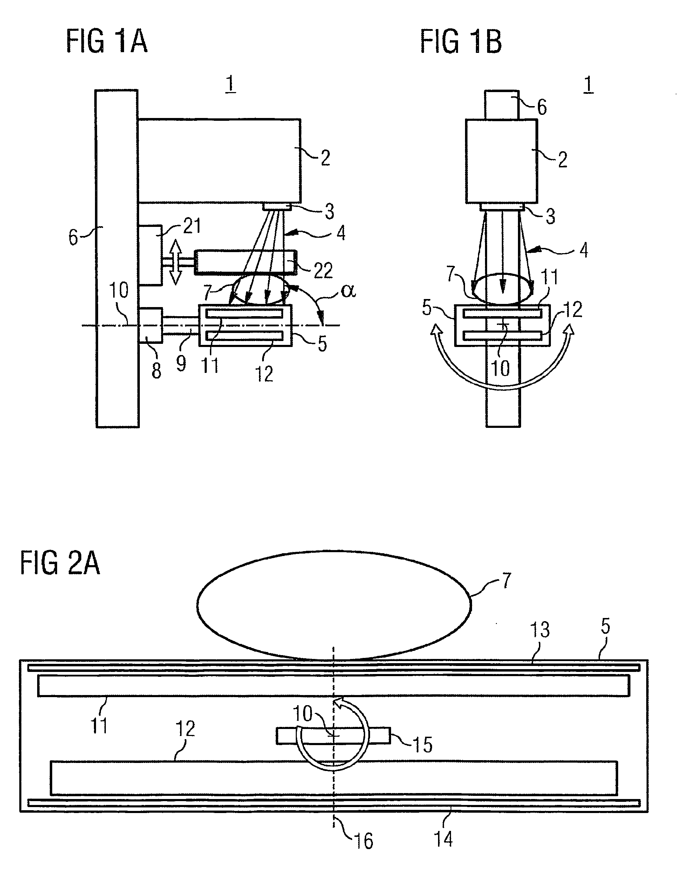

Die Figuren 1A und 1B zeigen schematisch ein Gerät zur medizinischen Bildgebung gemäß der vorliegenden Erfindung, das zur Gewinnung von Mammografiebildern ausgebildet ist.

Dabei zeigt Fig. 1A eine Seitenansicht und Fig. 1B eine Ansicht von vorne auf das erfindungsgemäße Gerät.

Das Gerät 1 weist eine von einem Kopf 2 getragene Strahlungsquelle 3 zur Emission einer Röntgenstrahlung 4 auf. Der Kopf 2 wird von einer Tragsäule 6 getragen. Unterhalb der Strahlungsquelle 3 ist eine Empfangseinrichtung zum Empfangen der von der Strahlungsquelle 3 emittierten Röntgenstrahlung 4 vorgesehen.

Die Empfangseinrichtung wird ebenfalls von der Tragsäule 6 getragen und weist gemäß der in den Figuren 1A und 1B gezeigten ersten besonders bevorzugten Ausführungsform einen Motor 8, einen von dem Motor 8 getragenen ersten Tragarm 9 sowie einen von dem ersten Tragarm 9 getragenen Träger 5 auf. Der Träger 5 ist in einem Strahlengang der Messstrahlung 4 angeordnet. In der vorliegenden Ausführungsform ist der Träger 5 aus Kohlenstofffasern gebildet.

Der Träger 5 kann alternative aber auch aus einem anderen Material wie z.B. Kunststoff gebildet sein. Dabei ist das Material vorzugsweise für die jeweilige Messstrahlung durchlässig.

Zwischen dem Träger 5 und der Strahlungsquelle 3 ist in dem Strahlungsgang der Röntgenstrahlung 4 ein Messbereich zum Anordnen eines Messobjektes 7 - im vorliegenden Fall einer weiblichen Brust - vorgesehen.

Wie aus den Figuren 1A und 1B ersichtlich, dient die jeweilige der Röntgenquelle 3 zugewandte Oberfläche des Trägers 5 gleichzeitig als Ablagefläche für das jeweilige Messobjekt (hier die weibliche Brust 7).

Weiter ist im Strahlengang der Röntgenstrahlung 4 oberhalb des Messobjektes 7 eine für die jeweilige Messstrahlung durchlässige Kompressionsplatte 22 vorgesehen. Im vorliegenden Ausführungsbeispiel ist die Kompressionsplatte 22 aus Kunststoff gebildet.

Die Kompressionsplatte 22 wird von einer Kompressionseinrichtung 21 getragen, die an der Tragsäule 6 des erfindungsgemäßen Gerätes befestigt ist. Durch eine von der Kompressionseinrichtung 21 bewirkte Vertikalbewegung der Kompressionsplatte 22 ist es möglich, das Messobjekt 7 zwischen der Kompressionsplatte 22 und der von dem Träger 5 gebildeten Ablagefläche zu komprimieren. Die Ablagefläche des Trägers 5 dient somit als untere Kompressionsfläche.

Der besseren Übersichtlichkeit wegen sind die Kompressionsplatte 22 und die Kompressionseinrichtung 21 in Fig. 1B nicht dargestellt.

Der Träger 5 ist mittels des Motors 8 über den ersten Tragarm 9 um eine Drehachse 10 schwenkbar. Dabei schließt die Drehachse 10 zu dem Strahlengang der Röntgenstrahlung 4 einen Winkel α von im wesentlichen 90° ein.

Alternativ ist es jedoch ausreichend, wenn der Winkel α zwischen 80° und 100°, vorzugsweise zwischen 85° und 95° und bevorzugt zwischen 88° und 92° beträgt.

Gemäß einer alternativen Ausführungsform erfolgt das Verschwenken des Trägers 5 um die Drehachse 10 nicht mittels des Motors 8 sondern manuell. Auf das Vorsehen des Motors 8 kann also gemäß dieser (in den Figuren nicht eigens gezeigten) Ausführungsform verzichtet werden.

Wie aus den Figuren 1A und 1B hervorgeht, ist die Drehachse 10 gemäß der besonders bevorzugten ersten Ausführungsform der vorliegenden Erfindung in Reihe mit dem Messbereich zentral im Strahlengang der Röntgenstrahlung 4 angeordnet.

Der Träger 5 trägt eine erste Empfangsfläche 11 für die Röntgenstrahlung 4 sowie eine zweite Empfangsfläche 12 für die Röntgenstrahlung 4.

Im vorliegenden Beispiel handelt es sich bei der ersten Empfangsfläche 11 um eine austauschbare Röntgenfilmkassette mit einem Röntgenfilm mit einer Aufnahmefläche von 18 x 24 cm oder 24 x 30 cm und bei der zweiten Empfangsfläche 12 um einen großflächigen niedrigauflösenden Festkörperdetektor (hier: Digitalsensor) für Röntgenstrahlung zur Erstellung von FFDM (Full Field Digital Mammographie) Bildern.

Alternativ zu der Röntgenfilmkassette mit einem Röntgenfilm kann auch ein CCD-Sensor eingesetzt werden, der in eine Röntgenfilmkassette eingebaut ist. Die Aufnahmefläche eines derartigen CCD-Sensors ist jedoch deutlich kleiner als die eines Röntgenfilms.

Wie aus den Figuren 1A und 1B deutlich hervorgeht, sind die Empfangsflächen 11 und 12 im Wesentlichen parallel zueinander und parallel zur Drehachse 10 des Trägers 5 angeordnet. Die Drehachse 10 ist in dieser Ausführungsform zwischen den Empfangsflächen 11 und 12 angeordnet.

Durch Verschwenken des Trägers 5 im Uhrzeigersinn oder gegen den Uhrzeigersinn um 180° um die Drehachse 10 ist es möglich, wahlweise die erste oder die zweite Empfangsfläche 11, 12 in eine Messstellung zu bewegen.

In der Messstellung ist die jeweilige Empfangsfläche 11 bzw. 12 benachbart zum Messbereich so im Strahlengang für die Röntgenstrahlung (Messstrahlung) 4 angeordnet, dass sie in Richtung zu der Strahlungsquelle 3 hin ausgerichtet ist. Weiter schließen der Strahlengang der Röntgenstrahlung 4 und eine in Messstellung befindliche Empfangsfläche 11 bzw. 12 einen Winkel von im Wesentlichen 90° ein.

Somit kommt durch Verschwenken des Trägers 5 wahlweise die erste oder die zweite Empfangsfläche 11 bzw. 12 auf der Oberseite des Trägers 5 zu liegen. Die Fixierung des Trägers 5 und damit die Fixierung der jeweiligen Empfangsfläche 11 und 12 in der jeweiligen Messstellung erfolgt in den Figuren 1A und 1B über den Motor 8. Alternativ kann jedoch eine separate Fixiereinrichtung, die vorzugsweise eine Rasterung aufweist, vorgesehen sein.

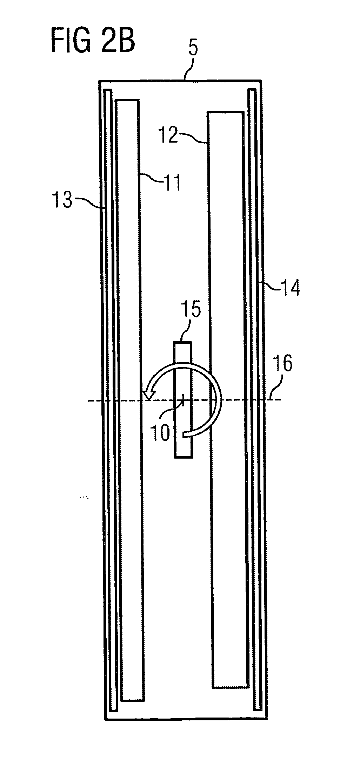

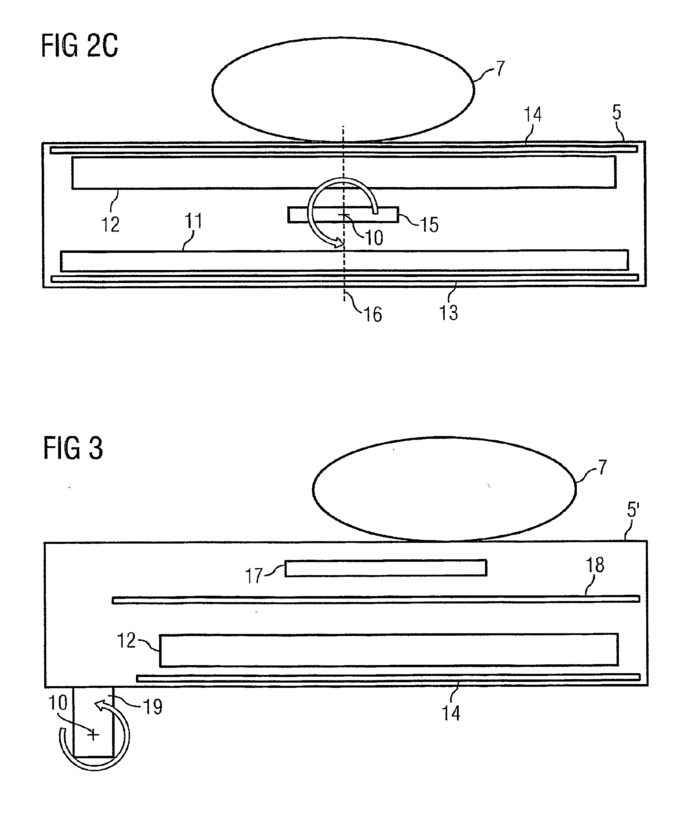

In den Figuren 2A, 2B und 2C wird der Träger 5 des in den Figuren 1A und 1B gezeigten erfindungsgemäßen Gerätes zur medizinischen Bildgebung 1 gemäß der ersten bevorzugten Ausführungsform der vorliegenden Erfindung in verschiedenen Schwenkstellungen dargestellt. Dabei handelt es sich bei den Figuren 2A, 2B und 2C um schematische Schnittansichten durch den Träger 5 senkrecht zur Drehachse 10.

Wie bereits erläutert, trägt der Träger 5 eine erste Empfangsfläche 11 in Form einer austauschbaren Röntgenfilmkassette, die einen Röntgenfilm oder einen CCD-Sensor enthalten kann. Weiter trägt der Träger 5 eine zweite Empfangsfläche 12 in Form eines niedrigauflösenden Digitalsensors für FFDM Aufnahmen.

Vor der Röntgenfilmkassette 11 (die an Stelle eines Röntgenfilmes auch einen CCD-Sensor enthalten kann) bzw. dem niedrigauflösenden Digitalsensor 12 sind ein erster bzw. ein zweiter Filter 13, 14 angeordnet. Im vorliegenden Beispiel handelt es sich bei den Filtern 13 und 14 um Streustrahlungsraster, die jeweils an den darunter liegenden Röntgenfilm 11 (oder CCD-Sensor) bzw. den darunter liegenden niedrigauflösenden Digitalsensor 12 angepasst sind.

Weiter trägt der in den Figuren 2A bis 2C gezeigte Träger 5 gemäß der ersten bevorzugten Ausführungsform eine Kontrolleinrichtung 15, die zur automatischen Kontrolle der empfangenen Röntgenstrahlung 4 dient. Dabei ist eine gemeinsame Kontrolleinrichtung 15 für die beiden Empfangsflächen 11 und 12 vorgesehen. Durch die Verwendung einer gemeinsamen Kontrolleinrichtung 15 kann die Zahl der für das erfinderische Gerät verwendeten Bauteile klein gehalten. Hierdurch können die Herstellungskosten reduziert werden.

Bei der Verwendung eines Röntgenfilmes in der Röntgenfilmkassette 11 übernimmt die Kontrolleinrichtung 15 vorzugsweise die Aufgabe eines AEC-Detektors (Automatic Exposure Control). Somit misst die Kontrolleinrichtung die empfangene Strahlung um die richtige bzw. optimale Belichtungszeit für den Röntgenfilm zu errechnen.

Bei der Verwendung eines CCD-Sensors in der Röntgenfilmkassette 11 oder bei Verwendung des niedrigauflösenden Digitalsensors 12 wird die richtige bzw. optimale Belichtungszeit automatisch von dem jeweiligen Sensor bestimmt. Ein zusätzlicher AEC-Detektor wird nicht benötigt. In diesem Fall kann die Kontrolleinrichtung 15 als zusätzliche Sicherheitseinrichtung dienen, um eine unnötige Strahlenbelastung bei einem Ausfall des CCD-Sensors oder des niedrigauflösenden Digitalsensors 12 zu vermeiden.

Wie aus den Figuren 2A bis 2C weiter hervorgeht, werden die Empfangsflächen 11 und 12 mit den dazugehörigen Filtern 13 und 14 so vom Träger 5 getragen, und ist die Drehachse 10 so angeordnet, dass eine senkrechte Projektion 16 der Drehachse 10 auf die jeweilige Empfangsfläche 11 bzw. 12 die jeweilige Empfangsfläche 11 bzw. 12 halbiert. Die senkrechte Projektion 16 wird in den Figuren durch eine gestrichelte Linie verdeutlicht.

Hierdurch wird sichergestellt, dass die jeweilige Empfangsfläche 11 bzw. 12 in der jeweiligen Messstellung jeweils mittig zur Drehachse 10 ausgerichtet ist, was die Bedienung durch einen Arzt besonders intuitiv und damit einfach macht.

Fig. 2A zeigt den Träger 5 in einer Stellung, in der er zur Erstellung eines analogen (Röntgenfilm-)Bildes der weiblichen Brust 7 unter Verwendung des austauschbaren Röntgenfilmes 11 und der Kontrolleinrichtung 15 geeignet ist.

Fig. 2B zeigt den Träger 5 in einer mittleren Schwenkstellung, bei der keine Erstellung eines Mammografiebildes möglich ist.

Fig. 2C zeigt den Träger 5 in einer Stellung, in der sich der niedrigauflösende Digitalsensor 12 in einer Messstellung befindet, so dass die Erstellung eines digitalen (FFDM-)Bildes der weiblichen Brust 7 möglich ist.

Fig. 3 zeigt eine alternative Ausführungsform einer Empfangseinrichtung des in den Figuren 1A und 1B gezeigten erfindungsgemäßen Gerätes zur medizinischen Bildgebung 1. Dabei handelt es sich bei der Figur 3 um eine schematische Schnittansicht senkrecht zur Drehachse 10.

Die in Fig. 3 gezeigte Empfangseinrichtung unterscheidet sich von der vorstehend beschriebenen besonders bevorzugten ersten Ausführungsform dadurch, dass der Träger 5' neben dem niedrigauflösenden Digitalsensor 12 zur Erstellung von FFDM Bildern und dem Streustrahlungsraster 14 einen CCD-Sensor 17 zur Erstellung von hochauflösenden Detailaufnahmen der weiblichen Brust 7 trägt. Dabei ist die Empfangsfläche des hochauflösenden CCD-Sensors 17 ersichtlich kleiner als die des niedrigauflösenden Digitalsensors 12.

Auch wenn in dieser Ausführungsform vor dem CCD-Sensor kein Streustrahlenraster vorgesehen ist, kann erforderlichenfalls vor dem CCD-Sensor zusätzlich ein Streustrahlenraster bereitgestellt werden.

Zwischen dem hochauflösenden CCD Sensor 17 und dem niedrigauflösenden Digitalsensor 12 ist eine Abschirmung 18 in Form einer Bleiplatte vorgesehen, die ebenfalls vom Träger 5' getragen wird. Die Abschirmung 18 schirmt die jeweils nicht in der Messstellung befindliche Empfangsfläche 17 bzw. 12 gegenüber dem Strahlengang der Röntgenstrahlung 4 ab und verhindert so, dass Röntgenstrahlung 4 auf der Unterseite des Trägers 5' austreten kann. Hierdurch wird die Strahlenbelastung für einen Patienten reduziert. Außerdem wird eine unbeabsichtigte Belichtung der nicht in Messstellung befindlichen Empfangsfläche 17 bzw. 12 sowie eine Strahlenbelastung dieser Empfangsfläche 17 bzw. 12 unterbunden.

Alternativ zu der Verwendung von Blei für die Abschirmung 18 kann bei Verwendung sehr weicher Röntgenstrahlung (ca. 20-35 kV) auch auf ein anderes für diese Röntgenstrahlung undurchlässiges Material zurückgegriffen werden. Dies ist aus Umweltschutzgesichtspunkten sinnvoll. Eine derartige weiche Röntgenstrahlung wird in der Mammographie üblicherweise verwendet.

Wie aus der Fig. 3 ersichtlich, ist die Drehachse 10 in dieser alternativen Ausführungsform nicht innerhalb des Trägers 5' zwischen den beiden Empfangsflächen 12 und 17 angeordnet, sondern außerhalb des Trägers 5'. Zur Verschwenkung des Trägers 5' um die Drehachse 10 ist daher die Verwendung eines zweiten Tragarms 19 vorgesehen.

Auch werden die Empfangsflächen 12 und 17 von einer jeweiligen senkrechten Projektion der Drehachse 10 in dieser Ausführungsform nicht halbiert.

Ein derartiger Aufbau ist sinnvoll, wenn die für die Drehachse 10 erforderliche Mechanik beispielsweise aus Platzgründen nicht innerhalb des Trägers vorgesehen werden kann.

Ein derartiger Aufbau hat den Nachteil, dass ein Verschwenken des Trägers 5' zu einer relativ ausladenden Bewegung führt und sich die beiden Empfangsflächen 12 und 17 in der jeweiligen Messstellung nicht am gleichen Ort sondern an sowohl horizontal als auch vertikal versetzten Orten befinden.

Dies kann das Vorsehen von in den Figuren nicht gezeigten horizontalen bzw. vertikalen Ausgleichseinrichtungen erforderlich machen, um die jeweilige Empfangsfläche jeweils im Fokus im Strahlengang der Röntgenstrahlung anzuordnen.

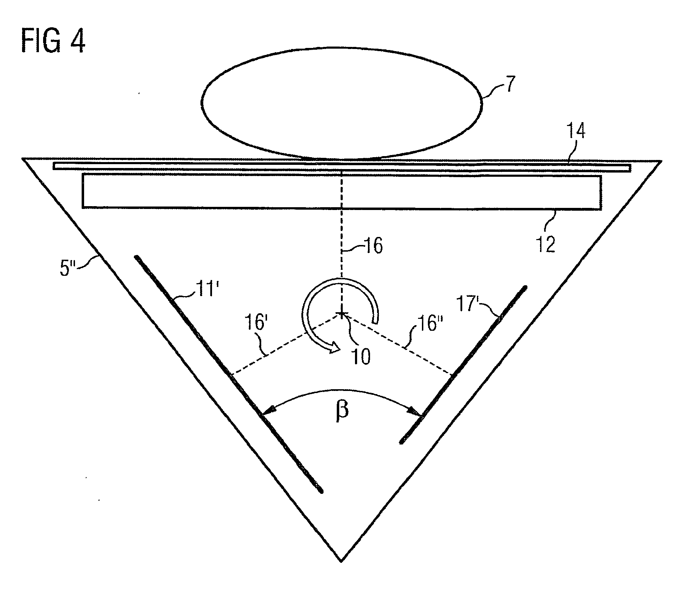

In Fig. 4 wird eine zweite bevorzugte Ausführungsform des Trägers 5" einer Empfangseinrichtung eines erfindungsgemäßen Geräts zur medizinischen Bildgebung 1 gezeigt. Auch Fig. 4 ist eine Querschnittsansicht durch den Träger 5" parallel zur Drehachse 10.

Der Träger 5" trägt eine Halterung zur Aufnahme eines austauschbaren Röntgenfilmes 11', einen großflächigen niedrigauflösenden Digitalsensor 12 zur Erstellung von FFDM Bildern, sowie einen kleinflächigen hochauflösenden CCD Sensor 17' zur Erstellung von Detailaufnahmen der weiblichen Brust 7. Über dem niedrigauflösenden Digitalsensor 12 ist ein Streustrahlungsraster 14 vorgesehen.

Es ist offensichtlich, dass ein entsprechend angepasster Filter auch über der Aufnahme für den austauschbaren Röntgenfilm 11' und/oder über dem hochauflösenden CCD Sensor 17' vorgesehen sein kann.

Wie aus der Fig. 4 ersichtlich, sind die Empfangsflächen 11', 12 und 17' im Wesentlichen parallel zur Drehachse 10 ausgerichtet. Eine senkrechte Projektion 16, 16', 16" der Drehachse 10 auf die Empfangsflächen 11', 12 und 17' halbiert die Empfangsflächen 11' und 12. Die Empfangsfläche 17' wird in der gezeigten Ausführungsform jedoch nicht halbiert.

Eine Anordnung, bei der eine senkrechte Projektion 16" der Drehachse 10 die Empfangsfläche 17' nicht halbiert ist insbesondere dann sinnvoll, wenn das Messobjekt 7 bei in Messstellung befindlicher Empfangsfläche 17' nicht mittig über der Drehachse 10 angeordnet werden soll oder kann.

Wie ersichtlich ist der Träger 5" so ausgebildet, dass die von ihm getragenen Empfangsflächen 11', 12 und 17' so angeordnet sind, dass benachbarte Empfangsflächen miteinander einen Winkel β von im Wesentlichen 60° einschließen.

Um ein Verschwenken des Trägers 5" mit einer möglichst wenig ausladenden Bewegung zu ermöglichen, ist die Drehachse 10 in der in Fig. 4 gezeigten Ausführungsform im geometrischen Flächenschwerpunkt des von dem Träger 5" für die Empfangsflächen 11', 12 und 17' umschlossenen Volumens angeordnet.

Auch wenn der in Fig. 4 gezeigte Pfeil ein Verschwenken des Trägers 5" entgegen dem Uhrzeigersinn um die Drehachse 10 herum andeutet, ist es offensichtlich, dass der Träger 5" nach Belieben auch im Uhrzeigersinn oder abwechselnd im Uhrzeigersinn und gegen den Uhrzeigersinn um die Drehachse 10 herum verschwenkt werden kann.

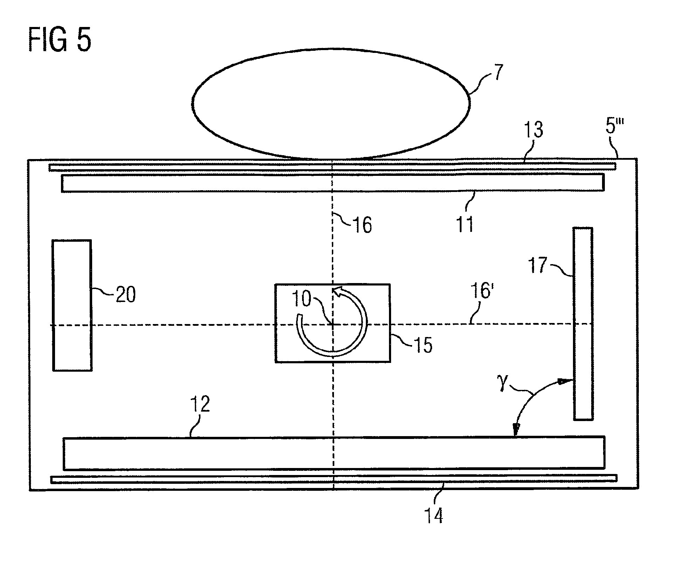

Fig. 5 zeigt eine dritte bevorzugte Ausführungsform eines Trägers 5"' der Empfangseinrichtung eines erfindungsgemäßen Gerätes zur medizinischen Bildgebung 1 zur Gewinnung von Mammografiebildern. Auch Fig. 5 ist eine schematische Querschnittsansicht durch den Träger 5"' parallel zur Drehachse 10.

Der Träger 5''' trägt einen austauschbaren Röntgenfilm 11, einen großflächigen niedrigauflösenden Digitalsensor 12 zur Erstellung von FFDM Bildern, einen kleinflächigen hochauflösenden CCD Sensor 17 zur Erstellung von Detailaufnahmen der weiblichen Brust 7 sowie einen Träger 20 für eine Lumineszenz-Radiografie-Folie. Vor dem austauschbaren Röntgenfilm 11 und dem niedrigauflösenden Digitalsensor 12 sind jeweils Streustrahlungsraster 13 bzw. 14 angeordnet.

Es ist offensichtlich, dass ein entsprechend angepasstes Streustrahlungsraster alternativ auch über dem kleinflächigen hochauflösenden CCD Sensor 17 sowie über dem Träger 20 für die Lumineszenz-Radiografie-Folie vorgesehen sein kann.

In der in Fig. 5 gezeigten Ausführungsform ist die Schwenkachse wiederum im geometrischen Flächenschwerpunkt des von dem Träger 5''' für die Empfangsflächen 11, 12, 17 und 20 umschlossenen Volumens angeordnet. Dabei sind die Empfangsflächen 11, 12 und 17 so angeordnet, dass senkrechte Projektionen 16 bzw. 16' auf die jeweiligen Empfangsflächen die Empfangsflächen halbieren. Allgemein schließen in dieser Ausführungsform benachbarte Empfangsflächen 11, 12, 17, 20 miteinander einen Winkel γ von im Wesentlichen 90° ein.

Somit ermöglicht es der in Fig. 5 gezeigte Träger 5"' gemäß der dritten bevorzugten Ausführungsform des erfindungsgemäßen Gerätes zur medizinischen Bildgebung 1 zur Gewinnung von Mammografiebildern, bei kompakter Bauart mittels vier verschiedener Messverfahren Mammografiebilder zu erstellen und somit das Messverfahren optimal an den jeweiligen Anwendungsfall anzupassen.

Auch wenn in den vorangehenden Beispielen lediglich die Verwendung von Röntgenfilmen, niedrigauflösenden Digitalsensoren / Detektoren für FFDM Aufnahmen, hochauflösenden CCD Sensoren für Detailaufnahmen sowie Lumineszenz-Radiografie-Folien als Empfangsflächen offenbart wurde, ist es offensichtlich, dass die Empfangsflächen durch alternative Messeinrichtungen realisiert werden können. Weiter kann der Träger der Empfangseinrichtung auch mehr als nur vier Empfangsflächen tragen.

Zusammenfassend ist es erfindungsgemäß möglich, ein Gerät zur medizinischen Bildgebung zur Erstellung von Mammografiebildern bereitzustellen, welches einen platzsparenden und mechanisch einfachen Aufbau aufweist und besonders robust ist.

## Patentansprüche

1. Gerät zur medizinischen Bildgebung (1), aufweisend eine Strahlungsquelle (3) zur Emission einer Messstrahlung (4), eine Empfangseinrichtung zum Empfangen der von der Strahlungsquelle (3) emittierten Messstrahlung (4), sowie einen Messbereich zum Anordnen eines Messobjektes (7), der in einem Strahlengang der Messstrahlung (4) zwischen Strahlungsquelle (3) und Empfangseinrichtung angeordnet ist,
wobei die Empfangseinrichtung einen um eine einzige Drehachse (10) schwenkbaren Träger (5; 5'; 5" ; 5"') aufweist, der wenigstens zwei Empfangsflächen (11, 12, 17, 20; 11', 17') für die Messstrahlung (4) trägt und die Empfangsflächen (11, 12, 17, 20; 11', 17') abwechselnd in eine Messstellung bewegen kann, wobei die Empfgangsflächen (11, 12, 17, 20; 11', 17') parallel zur Drehachse (10) des Trägers (5; 5'; 5"; 5"') angeordnet sind und
die Drehachse (10) des Trägers (5; 5'; 5"; 5"') zu dem Strahlengang der Messstrahlung (4) im Wesentlichen senkrecht steht,
**dadurch gekennzeichnet, dass** die Drehachse (10) bezüglich dem Messbereich und der sich jeweils in der Messstellung befindlichen Empfangsfläche (11, 12, 17, 20; 11', 17') in die der Strahlungsquelle (3) abgewandte Richtung versetzt ist und die Empfangsflächen (11, 12, 17, 20; 11', 17') der Drehachse (10) abgewandt sind.

2. Gerät zur medizinischen Bildgebung (1) nach Anspruch 1,
**dadurch gekennzeichnet, dass** die Drehachse (10) mit dem Strahlengang der Messstrahlung (4) einen Winkel (α) von zwischen 80° und 100°, vorzugsweise zwischen 85° und 95°, bevorzugt zwischen 88° und 92° und besonders bevorzugt von 90° einschließt.

3. Gerät zur medizinischen Bildgebung (1) nach Anspruch 1 oder 2,
**dadurch gekennzeichnet, dass** die Drehachse (10) zwischen den Empfangsflächen (11, 12, 17, 20; 11', 17') angeordnet ist.

4. Gerät zur medizinischen Bildgebung (1) nach einem der Ansprüche 1, 2 oder 3,
**dadurch gekennzeichnet, dass** die Empfangsflächen (11, 12, 17; 11') von einer senkrechten Projektion (16, 16', 16") der Drehachse (10) jeweils im Wesentlichen halbiert werden.

5. Gerät zur medizinischen Bildgebung (1) nach einem der vorangegangenen Ansprüche,
**dadurch gekennzeichnet, dass** die Drehachse (10) zentral im Strahlengang der Messstrahlung (4) angeordnet ist.

6. Gerät zur medizinischen Bildgebung (1) nach einem der vorangegangenen Ansprüche,
**dadurch gekennzeichnet, dass** die Drehachse (10) im geometrischen Schwerpunkt eines vom Träger (5, 5", 5"') für die Empfangsflächen (11, 12, 17, 20; 11', 17') umschlossenen Volumens angeordnet ist.

7. Gerät zur medizinischen Bildgebung (1) nach einem der vorangegangenen Ansprüche,
**dadurch gekennzeichnet, dass** der Träger (5; 5'; 5"; 5"') gleichzeitig eine Ablagefläche für das Messobjekt (7) aufweist.

8. Gerät zur medizinischen Bildgebung (1) nach einem der vorangegangenen Ansprüche,
**dadurch gekennzeichnet, dass** zwischen den Empfangsflächen (11, 12, 17, 20) eine vom Träger (5; 5"') getragene Kontrolleinrichtung (15) zur Kontrolle der empfangenen Messstrahlung (4) vorgesehen ist.

9. Gerät zur medizinischen Bildgebung (1) nach einem der vorangegangenen Ansprüche,
**dadurch gekennzeichnet, dass** der Träger (5') zusätzlich wenigstens eine Abschirmung (18) trägt, die für nicht in der Messstellung befindliche Empfangsflächen (12, 17) im Strahlengang der Messstrahlung (4) angeordnet ist.

10. Gerät zur medizinischen Bildgebung (1) nach einem der vorangegangenen Ansprüche,
**dadurch gekennzeichnet, dass** der Träger (5; 5') zwei im Wesentlichen parallele Empfangsflächen (11, 12; 12, 17) für die Messstrahlung (4) trägt.

11. Gerät zur medizinischen Bildgebung (1) nach einem der Ansprüche 1 bis 9,
**dadurch gekennzeichnet, dass** der Träger (5") drei Empfangsflächen (11', 12, 17') für die Messstrahlung (4) trägt, wobei benachbarte Empfangsflächen (11', 12, 17') miteinander einen Winkel (β) von im Wesentlichen 60° einschließen.

12. Gerät zur medizinischen Bildgebung (1) nach einem der Ansprüche 1 bis 9,
**dadurch gekennzeichnet, dass** der Träger (5"') vier Empfangsflächen (11, 12, 17, 20) für die Messstrahlung (4) trägt, wobei benachbarte Empfangsflächen (11, 12, 17, 20) miteinander einen Winkel (γ) von im Wesentlichen 90° einschließen.

13. Gerät zur medizinischen Bildgebung (1) nach einem der vorangegangenen Ansprüche,
**dadurch gekennzeichnet, dass** die von der Strahlungsquelle (3) emittierte Messstrahlung (4) eine Röntgenstrahlung ist.

14. Gerät zur medizinischen Bildgebung (1) nach Anspruch 13,
**dadurch gekennzeichnet, dass** wenigstens eine der Empfangsflächen (17, 17') ein Festkörperdetektor für Röntgenstrahlung und vorzugsweise ein CCD-Sensor ist.

15. Gerät zur medizinischen Bildgebung (1) nach Anspruch 13 oder 14,
**dadurch gekennzeichnet, dass** wenigstens eine der Empfangsflächen (11, 11') ein Röntgenfilm ist.

16. Gerät zur medizinischen Bildgebung (1) nach Anspruch 13, 14 oder 15,
**dadurch gekennzeichnet, dass** wenigstens eine der Empfangsflächen (20) eine Lumineszenz-Radiographie-Folie ist.

17. Gerät zur medizinischen Bildgebung (1) nach einem der vorangegangenen Ansprüche,
**dadurch gekennzeichnet, dass** das Gerät zur medizinischen Bildgebung (1) zur Gewinnung von Mammografiebildern ausgebildet ist.

## Claims

1. Medical imaging equipment (1), comprising a radiation source (3) for emitting measuring radiation (4),
a receiving device for receiving the measuring radiation (4) that is emitted by the radiation source (3), and a measuring region for arranging an object for measurement (7), which is situated in a beam path of the measuring radiation (4) between the radiation source (3) and the receiving device,
wherein the receiving device has a support (5; 5'; 5''; 5"') which can be pivoted about a single rotational axis (10), said support carrying at least two receiving surfaces (11, 12, 17, 20; 11', 17') for the measuring radiation (4) and being able to move the receiving surfaces (11, 12, 17, 20; 11', 17') alternately into a measuring position,
with the receiving surfaces (11, 12, 17, 20; 11', 17') being arranged parallel to the rotational axis (10) of the support (5; 5'; 5"; 5"') and
the rotational axis (10) of the support (5; 5'; 5"; 5"') being perpendicular to the beam path of the measuring radiation (4),
**characterised in that**
the rotational axis (10) is moved in the direction facing away from the radiation source (3) in respect of the measuring region and the receiving surfaces (11, 12, 17, 20; 11', 17') located in the measuring position and the receiving surfaces (11, 12, 17, 20; 11', 17') are facing away from the rotational axis (10).

2. Medical imaging equipment (1) according to claim 1, **characterised in that** the rotational axis (10) together with the beam path of the measuring radiation (4) encompasses an angle (α) of between 80° and 100°, preferably between 85° and 95°, preferably between 88° and 92° and particularly preferably of 90°.

3. Medical imaging equipment (1) according to claim 1 or 2,
**characterised in that** the rotational axis (10) is arranged between the receiving surfaces (11, 12, 17, 20; 11', 17').

4. Medical imaging equipment (1) according to one of the claims 1, 2 or 3,
**characterised in that** the receiving surfaces (11, 12, 17; 11') are each essentially bisected by a perpendicular projection (16, 16', 16") of the rotational axis (10).

5. Medical imaging equipment (1) according to one of the preceding claims,
**characterised in that** the rotational axis (10) is arranged centrally in the beam path of the measuring radiation (4).

6. Medical imaging equipment (1) according to one of the preceding claims,
**characterised in that** the rotational axis (10) is arranged in the geometric centre of gravity of a volume encompassed by the support (5; 5"; 5''') for the receiving surfaces (11, 12, 17, 20; 11', 17').

7. Medical imaging equipment (1) according to one of the preceding claims,
**characterised in that** at the same time the support (5; 5'; 5"; 5"') has a deposit surface for the object for measurement (7).

8. Medical imaging equipment (1) according to one of the preceding claims,
**characterised in that** between the receiving surfaces (11, 12, 17, 20) there is a control device (15) carried by the support (5; 5"') for controlling the received measuring radiation (4).

9. Medical imaging equipment (1) according to one of the preceding claims,
**characterised in that** the support (5') additionally carries at least one shield (18), which is arranged in the beam path of the measuring radiation (4) for receiving surfaces (12, 17) that are not located in the measuring position.

10. Medical imaging equipment (1) according to one of the preceding claims,
**characterised in that** the support (5; 5') carries two essentially parallel receiving surfaces (11, 12; 12, 17) for the measuring radiation (4).

11. Medical imaging equipment (1) according to one of claims 1 to 9,
**characterised in that** the support (5") carries three receiving surfaces (11', 12, 17') for the measuring radiation (4), wherein adjacent receiving surfaces (11', 12, 17') encompass an angle (β) of essentially 60° with each other.

12. Medical imaging equipment (1) according to one of the claims 1 to 9,
**characterised in that** the support (5"') carries four receiving surfaces (11, 12, 17, 20) for the measuring radiation (4), wherein adjacent receiving surfaces (11, 12, 17, 20) encompass an angle (γ) of essentially 90° with each other.

13. Medical imaging equipment (1) according to one of the preceding claims,
**characterised in that** the measuring radiation (4) emitted by the radiation source (3) are x-rays.

14. Medical imaging equipment (1) according to claim 13,
**characterised in that** at least one of the receiving surfaces (17, 17') is a solid state detector for x-rays and preferably a CCD sensor.

15. Medical imaging equipment (1) according to claim 13 or 14,
**characterised in that** at least one of the receiving surfaces (11, 11') is an x-ray film.

16. Medical imaging equipment (1) according to claim 13, 14 or 15,
**characterised in that** at least one of the receiving surfaces (20) is a luminescence radiography screen.

17. Medical imaging equipment (1) according to one of the previous claims,
**characterised in that** the medical imaging equipment (1) is designed to obtain mammographic images.

## Revendications

1. Appareil pour la génération d'images médicales (1), présentant une source de rayonnement (3) pour l'émission d'un rayonnement de mesure (4), un dispositif de réception pour la réception du rayonnement de mesure (4) émis par la source de rayonnement (3) et une zone de mesure pour la mise en place d'un objet à mesurer (7), qui est disposée dans une trajectoire de faisceau du rayonnement de mesure (4) entre la source de rayonnement (3) et le dispositif de réception,
le dispositif de réception présentant un support (5 ; 5' ; 5"; 5"') pouvant basculer autour d'un unique axe de rotation (10), qui porte au moins deux surfaces de réception (11, 12, 17, 20 ; 11', 17') pour le rayonnement de mesure (4) et peut déplacer les surfaces de réception (11, 12, 17, 20 ; 11', 17') en alternance dans une position de mesure, les surfaces de réception (11, 12, 17, 20 ; 11', 17') étant disposées parallèlement à l'axe de rotation (10) du support (5 ; 5' ; 5"; 5"') et
l'axe de rotation (10) du support (5 ; 5' ; 5" ; 5"') étant sensiblement perpendiculaire à la trajectoire de faisceau du rayonnement de mesure (4),
**caractérisé en ce que**
l'axe de rotation (10) est décalé par rapport à la zone de mesure et la surface de réception (11, 12, 17, 20 ; 11', 17') se trouvant respectivement dans la position de mesure dans la direction détournée de la source de rayonnement (3) et les surfaces de réception (11, 12, 17, 20 ; 11', 17') sont détournées de l'axe de rotation (10).

2. Appareil pour la génération d'images médicales (1) selon la revendication 1, **caractérisé en ce que** l'axe de rotation (10) forme avec la trajectoire du faisceau du rayonnement de mesure (4) un angle (α) compris entre 80° et 100°, de préférence entre 85° et 95°, de préférence entre 88° et 92° et avec une préférence particulière de 90°.

3. Appareil pour la génération d'images médicales (1) selon la revendication 1 ou 2, **caractérisé en ce que** l'axe de rotation (10) est disposé entre les surfaces de réception (11, 12, 17, 20 ; 11' , 17').

4. Appareil pour la génération d'images médicales (1) selon l'une quelconque des revendications 1, 2 ou 3,
**caractérisé en ce que** les surfaces de réception (11, 12, 17; 11') sont divisées sensiblement en deux par une projection perpendiculaire (16, 16', 16") de l'axe de rotation (10).

5. Appareil pour la génération d'images médicales (1) selon l'une quelconque des revendications précédentes,
**caractérisé en ce que** l'axe de rotation (10) est disposé centralement dans la trajectoire du faisceau de rayonnement de mesure (4).

6. Appareil pour la génération d'images médicales (1) selon l'une quelconque des revendications précédentes,
**caractérisé en ce que** l'axe de rotation (10) est disposé au centre de gravité géométrique d'un volume entouré par le support (5, 5", 5"') pour les surfaces de réception (11, 12, 17, 20 ; 11', 17').

7. Appareil pour la génération d'images médicales (1) selon l'une quelconque des revendications précédentes,
**caractérisé en ce que** le support (5 ; 5' ; 5"'; 53') présente en même temps une surface de dépose pour l'objet à mesurer (7).

8. Appareil pour la génération d'images médicales (1) selon l'une quelconque des revendications précédentes,
**caractérisé en ce qu'**un dispositif de contrôle (15) porté par le support (5 ; 5"') est prévu entre les surfaces de réception (11, 12, 17, 20) pour le contrôle du rayonnement de mesure (4) reçu.

9. Appareil pour la génération d'images médicales (1) selon l'une quelconque des revendications précédentes,
**caractérisé en ce que** le support (5') porte en supplément au moins un blindage (18), qui est disposé dans la trajectoire de faisceau du rayonnement de mesure (4) pour des surfaces de réception (12, 17) ne se trouvant pas dans la position de mesure.

10. Appareil pour la génération d'images médicales (1) selon l'une quelconque des revendications précédentes,
**caractérisé en ce que** le support (5 ; 5') porte deux surfaces de réception (11, 12 ; 12, 17) sensiblement parallèles pour le rayonnement de mesure (4).

11. Appareil pour la génération d'images médicales (1) selon l'une quelconque des revendications 1 à 9,
**caractérisé en ce que** le support (5") porte trois surfaces de réception (11', 12, 17') pour le rayonnement de mesure (4), des surfaces de réception (11', 12, 17') voisines formant les unes avec les autres un angle (β) de sensiblement 60°.

12. Appareil pour la génération d'images médicales (1) selon l'une quelconque des revendications 1 à 9,
**caractérisé en ce que** le support (5"') porte quatre surfaces de réception (11, 12, 17, 20) pour le rayonnement de mesure (4), des surfaces de réception (11, 12, 17, 20) voisines formant les unes avec les autres un angle (γ) de sensiblement 90°.

13. Appareil pour la génération d'images médicales (1) selon l'une quelconque des revendications précédentes,
**caractérisé en ce que** le rayonnement de mesure (4) émis par la source de rayonnement (3) est un rayonnement X.

14. Appareil pour la génération d'images médicales (1) selon la revendication 13,
**caractérisé en ce qu'**au moins l'une des surfaces de réception (17, 17') est un détecteur à corps solide pour le rayonnement X et de préférence un capteur CCD.

15. Appareil pour la génération d'images médicales (1) selon la revendication 13 ou 14,
**caractérisé en ce qu'**au moins l'une des surfaces de réception (11, 11') est un film pour rayons X.

16. Appareil pour la génération d'images médicales (1) selon la revendication 13, 14 ou 15,
**caractérisé en ce qu'**au moins l'une des surfaces de réception (20) est un film de radiographie à luminescence.

17. Appareil pour la génération d'images médicales (1) selon l'une quelconque des revendications précédentes,
**caractérisé en ce que** l'appareil pour la génération d'images médicales (1) est conçu pour l'obtention d'images de mammographie.
